# EUROPEAN PATENT APPLICATION

(11) **EP 4 837 971 A1**
(43) Date of publication of application: **11.11.2026**
(21) Application number: 26177272.7
(22) Date of filing: 07.05.2026
(51) Int. Cl.: C12Q 1/6816

(54) **ESTIMATION METHOD**

(30) Priority: 08.05.2025 JP 2025077890; 30.04.2026 JP 2026076713
(71) Applicant: Canon Kabushiki Kaisha, Tokyo 146-8501 (JP)
(72) Inventor: FUJITA, Keigo, Tokyo 146-8501 (JP); TAMURA, Tsuruki, Tokyo 146-8501 (JP); SATO, Suguru, Tokyo 146-8501 (JP); OE, Suzu, Tokyo 146-8501 (JP)
(74) Representative: Marks & Clerk LLP

(57) **Abstract**

An estimation method of estimating at least one of whether there is at least one of linear DNA and circular DNA in a specimen, the proportion of at least one of linear DNA and circular DNA in DNA in the specimen, and the amount of at least one of linear DNA and circular DNA in the specimen includes raising or lowering a temperature of a specimen including DNA, detecting a signal varying with transition between a double-stranded state and a single-stranded state of DNA, and performing estimation on the basis of the signal.

## Description

### FIELD

### Field of the Invention

The present invention relates to an estimation method.

### BACKGROUND

In the related art, it is known that transformation efficiency varies depending on whether plasmid vector DNA maintains a circular shape or forms a linear shape due to physical shearing or the like when a plasmid vector is introduced into a bacterium or the like for phenotype transformation (or transfection). As the proportion of linear DNA becomes higher and the proportion of circular DNA becomes lower, the phenotype transformation efficiency becomes lower.

As well as a situation in which a plasmid vector is introduced, there is a situation in which it is important to ascertain whether there is linear DNA or circular DNA, the ratio therebetween (that is, proportions of the amount of linear DNA and the amount of circular DNA), or amounts thereof.

An example of the method of ascertaining whether there is linear DNA and/or circular DNA in a specimen including DNA or the ratio or amounts thereof in the related art is electrophoresis of DNA. When electrophoresis of DNA is performed, the electrophoresis speed on a negative electrode side differs between linear DNA and circular DNA, and thus it is possible to discriminate between circular DNA and linear DNA by positions of bands and to ascertain the ratio therebetween.

However, in the method of ascertaining whether there is linear DNA and/or circular DNA or the ratio or amounts thereof using electrophoresis, a time of at least 30 minutes is required until a result is obtained. In addition, there has been a problem that confirmation requires significant time and effort because electrophoresis of DNA requires many reagents such as gels and buffers, and it is also necessary to previously estimate the positions of bands for linear DNA and circular DNA.

The problem to be solved by embodiments disclosed in this specification and the drawings is to more quickly and/or easily estimate at least one of whether there is at least one of linear DNA and circular DNA in the specimen, a proportion of at least one of linear DNA and circular DNA in the DNA in the specimen, and an amount of at least one of linear DNA and circular DNA in the specimen, compared to conventional methods. However, the problem to be solved by the embodiments disclosed in this specification and the drawings are not limited to the above problem. Problems corresponding to the effects of each configuration shown in the embodiments described later can also be positioned as other problems.

An estimation method of an embodiment includes raising or lowering a temperature of a specimen including DNA and detecting a signal varying with transition between a double-stranded state and a single-stranded state of DNA and estimating at least one of whether there is at least one of linear DNA and circular DNA in the specimen, a proportion of at least one of linear DNA and circular DNA in the DNA in the specimen, and an amount of at least one of linear DNA and circular DNA in the specimen on the basis of the signal.

The signal may be based on fluorescence intensity from a DNA intercalator that emits fluorescence when the DNA intercalator is inserted between double strands of DNA.

The signal may be change of the fluorescence intensity per unit temperature change. The signal may be detected by raising the temperature of the specimen.

Whether there is circular DNA or a proportion thereof may be estimated on the basis of the signal.
when a drastic increase and a drastic decrease of the change do not occur, it may indicate that only circular DNA is included in the specimen.

When at least one of a drastic increase and a drastic decrease of the change occurs, it may indicate that linear DNA is included in the specimen.

When a temperature range in which at least one of the amount of increase and the amount of decrease of the change is equal to or higher than a predetermined value is included, it may indicate that linear DNA is included in the specimen. When a temperature range in which at least one of the amount of increase and the amount of decrease of the change is equal to or higher than the predetermined value is not included, it may indicate that circular DNA is included and linear DNA is not included in the specimen.

The amount of increase may be the amount of increase of the change in a range of 85°C to 93°C. The amount of decrease may be the amount of decrease of the change in a range of 80°C to 85°C.

A larger amount of increase or a larger amount of decrease of the change may indicate a higher proportion of linear DNA and a lower proportion of circular DNA in the DNA in the specimen.

The DNA included in the specimen may be at least one kind of DNA selected from a group consisting of plasmid DNA, mitochondrial DNA, eccDNA, and ecDNA.

An estimation method may include estimating an absolute amount of linear DNA in the specimen by multiplying an amount of DNA included in the specimen by the proportion of linear DNA estimated using the estimation method above.

An estimation method may include estimating an absolute amount of circular DNA in the specimen by multiplying an amount of DNA included in the specimen by the proportion of circular DNA estimated using the estimation method above.

### BRIEF DESCRIPTION OF THE DRAWINGS

FIG. 1 is a diagram schematically illustrating the high thermal stability of circular DNA with respect to linear DNA.
FIG. 2 is a graph illustrating a virtual melting curve in a sample in which the proportion of circular DNA is 100% which has been prepared on the basis of a melting curve of a sample including linear DNA.
FIG. 3 is a graph illustrating a relationship between fluorescence intensity and a temperature when a sample in which the proportion of circular DNA is 100% and a sample in which the proportion of linear DNA is 100% were heated.
FIG. 4 is a graph illustrating a melting curve when a sample in which the proportion of circular DNA is 100% and a sample in which the proportion of linear DNA is 100% were heated.
FIG. 5 is a graph illustrating melting curves prepared by heating a plurality of samples in which the ratio between linear DNA and circular DNA was varied.
FIG. 6 is a calibration curve prepared by plotting the proportion of circular DNA with respect to the amount of increase of a change of fluorescence intensity per unit temperature change when a plurality of samples in which the ratio between linear DNA and circular DNA was varied were heated.
FIG. 7 is a graph illustrating the height of change of fluorescence intensity per unit temperature change in a temperature range of 89°C to 90°C in the melting curve illustrated in FIG. 5 using an arrow.
FIG. 8 is a calibration curve prepared by plotting the proportion of circular DNA with respect to the height of change of fluorescence intensity per unit temperature change in a temperature range of 89°C to 90°C

### DETAILED DESCRIPTION

Hereinafter an exemplary embodiment of the present invention will be described in detail with reference to the accompanying drawings. The present invention is not limited to the following embodiment.

An estimation method according to an exemplary embodiment of the present invention is an estimation method including raising or lowering the temperature of a specimen including DNA, detecting a signal (hereinafter also referred to as a "transition change signal") varying with transition (shift) between a double-stranded state and a single-stranded state of DNA, and estimating at least one of whether there is at least one of linear DNA and circular DNA in the specimen, the proportion of at least one of the linear DNA and the circular DNA in the DNA in the specimen, and the amount of at least one of the linear DNA and the circular DNA in the specimen on the basis of the transition change signal.

With the estimation method according to the present embodiment, it is possible to simply determine whether there is linear DNA in a specimen, to simply measure whether there is circular DNA in the specimen, to simply measure the proportion of linear DNA in the DNA included in the specimen, to simply measure the proportion of circular DNA in the DNA included in the specimen, to simply measure proportions of the circular DNA and the linear DNA, to simply measure the amount of linear DNA in the specimen, and to simply measure the amount of circular DNA in the specimen. Accordingly, the estimation method according to the present embodiment can be understood as a simple measuring method. The amount of DNA in the specimen is a sum of the amount of the linear DNA in the specimen and the amount of the circular DNA in the specimen.

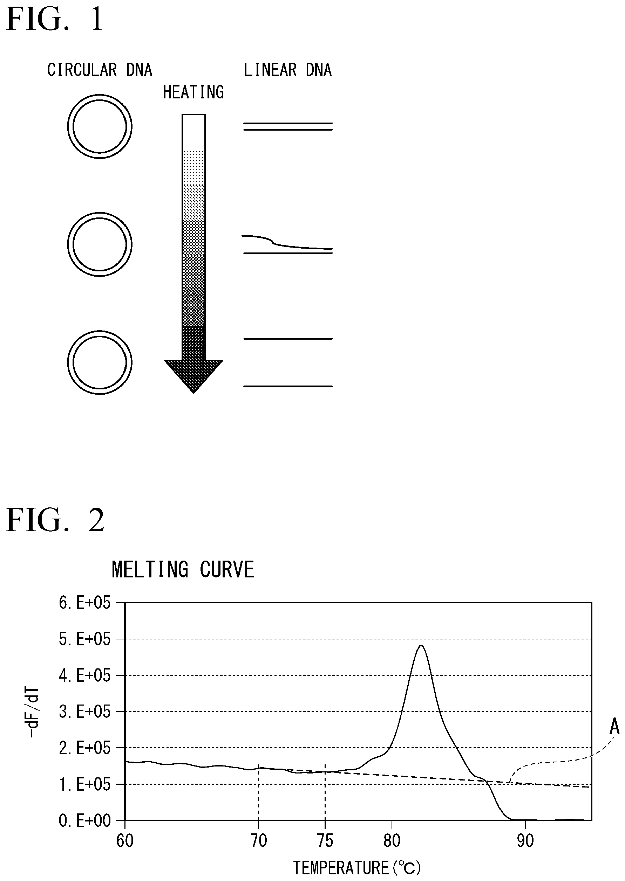

FIG. 1 is a diagram schematically illustrating the high thermal stability of circular DNA with respect to linear DNA.

The inventors of the present invention found that, when a melting curve (a dissociation curve) while raising or lowering the temperature of a specimen including DNA is drawn under the presence of DNA intercalator as will be described later in detail in examples and linear DNA are included in the specimen, the differential value of fluorescence intensity vs temperature drastically increases and decreases in the vicinity of a temperature at which linear double-stranded DNA dissociate and the amount of increase and the amount of decrease become larger as the proportion of linear DNA in the DNA included in the specimen increases. It was not previously known that the shape of the melting curve varied greatly between linear DNA and circular DNA.

This phenomenon occurs because linear DNA is lower in thermal stability than circular DNA and a double-stranded state is more easily denatured (dissociates) to a single-stranded state when they are heated. The differential value of fluorescence intensity vs temperature is a differential value dF/dT of the fluorescence intensity "F" at a temperature "T" or a negative differential value -dF/dT (which is namely a change of the fluorescence intensity per unit temperature change). dF denotes a detected change of fluorescence intensity, and dT denotes a change in temperature. "Per unit temperature change" in this specification is, for example, per 0.1°C, per 0.2°C, or 0.3°C, but is not limited thereto and can be appropriately set by a person skilled in the art.

### <Kind of specimen>

A specimen in which whether there is linear DNA and/or circular DNA or proportions thereof are estimated in the method according to the present embodiment is not particularly limited in kind as long as the specimen is a liquid including DNA. Preferably, the specimen is an aqueous solution including DNA.

DNA included in a specimen may be, for example, at least one kind of DNA selected from a group consisting of plasmid DNA, mitochondrial DNA, extrachromosomal circular DNA (eccDNA), and extrachromosomal DNA (ecDNA), and the kinds (origins) of DNA included in the specimen are not particularly limited. The kinds of circular DNA and linear DNA included in the specimen are not necessarily the same, and, for example, the circular DNA may be mitochondrial DNA and the linear DNA may be a nuclear DNA.

### <Kind of transition change signal>

The kind of the transition change signal varying with transition between a double-stranded state and a single-stranded state of DNA by raising or lowering the temperature of the specimen is not particularly limited, and the transition change signal may be a signal based on fluorescence intensity from a DNA intercalator emitting fluorescence when it is inserted between double strands of DNA as in Experimental Example 1 which will be described later in detail, may be a change of fluorescence intensity per unit temperature change as in Experimental Example 2, may be an absorbance, or may be a measurement result acquired by differential scanning calorimetry (DSC).

The change of fluorescence intensity per unit temperature change may be the aforementioned differential value vs temperature (dF/dT) or may be a negative differential value vs temperature (-dF/dT) obtained by multiplying the differential value dF/dT by -1.

The fluorescence intensity or the transition change signal which is a change of the fluorescence intensity per unit temperature change can be detected, for example, using a real-time PCR system or a fluorescence micro plate reader in which temperature control is possible, but is not limited thereto.

When the temperature of a specimen is raised or lowered in detecting the transition change signal, a raising speed or a lowering speed of the temperature (such as 0.1°C/second) is preferably constant. Accordingly, it is possible to enable more accurate estimation.

When the temperature of a specimen is raised, it is preferable to raise the temperature from a lower temperature than a temperature range in which linear DNA dissociates to a higher temperature than the temperature range in which linear DNA dissociates. When the temperature of a specimen is lowered, it is preferable to lower the temperature from a higher temperature than the temperature range in which linear DNA dissociates to a lower temperature than the temperature range in which linear DNA dissociates.

### (Signal of fluorescence intensity)

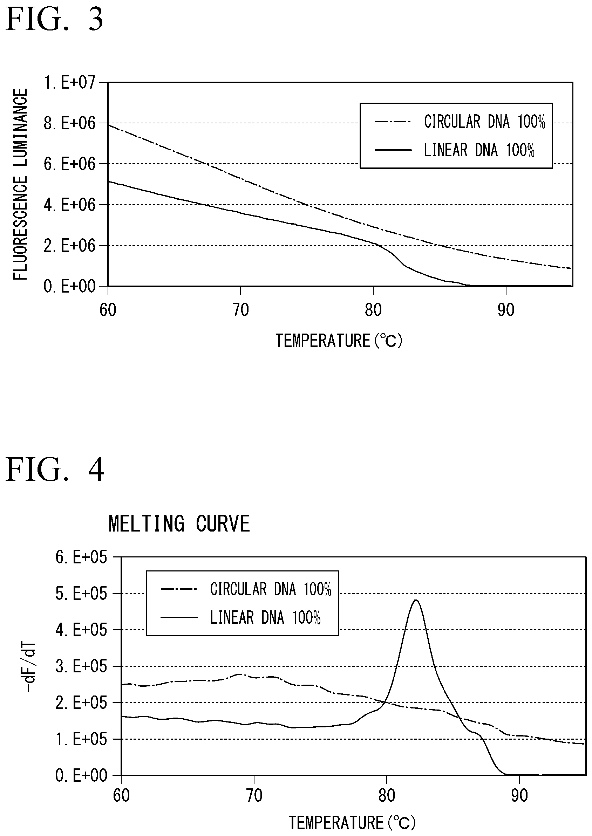

As will be described later in Experimental Example 1, when there is linear DNA in the specimen and the temperature of the specimen is raised, the fluorescence intensity from the DNA intercalator decreases greatly and drastically in the vicinity of the temperature range in which linear DNA dissociates (particularly see FIG. 3). On the other hand, when circular DNA accounts for 100% of the DNA in the specimen, the fluorescence intensity from the DNA intercalator decreases gradually with the raising of temperature. When the temperature of the specimen is raised, the fluorescence intensity decreases greatly and drastically as the proportion of linear DNA becomes higher and the proportion of circular DNA becomes lower.

Accordingly, by ascertaining change of the fluorescence intensity from a DNA intercalator when the temperature of the specimen is raised at a constant speed, it is possible to estimate one or more of whether there is linear double-stranded DNA and/or circular double-stranded DNA in the DNA of the specimen, substantial proportions (or the ratio) thereof, and amounts thereof.

When there is linear DNA in the specimen and the temperature of the specimen is lowered, the fluorescence intensity from the DNA intercalator increases drastically in the vicinity of the temperature range in which linear DNA dissociates. On the other hand, when circular DNA accounts for 100% of the DNA in the specimen, the fluorescence intensity from the DNA intercalator increases gradually with the lowering of temperature. When the temperature of the specimen is lowered, the fluorescence intensity increases drastically as the proportion of linear DNA becomes higher and the proportion of circular DNA becomes lower.

Accordingly, by ascertaining change of the fluorescence intensity from a DNA intercalator when the temperature of the specimen is lowered, it is possible to estimate one or more of whether there is linear double-stranded DNA and/or circular double-stranded DNA in the DNA of the specimen, substantial proportions (or the ratio) thereof, and amounts thereof.

When a DNA intercalator is used in the estimation method according to the present embodiment, the kind of the DNA intercalator is not particularly limited, and, for example, SYBR^{™} Green I (ThermoFisher Scientific) or EvaGreen^{™} (Biotium) can be appropriately used.

### (Signal of change of fluorescence intensity per unit temperature change)

As will be described later in Experimental Examples 1 and 2, when the temperature of the specimen is raised and there is linear DNA in the specimen, a change of fluorescence intensity per unit temperature change dF/dT or -dF/dT from a DNA intercalator which emits fluorescence when the DNA intercalator is inserted between double strands of DNA increases or decreases drastically in the vicinity of a temperature range in which linear DNA dissociates (particularly, see FIGS. 4, 5, and 7). On the other hand, when circular DNA accounts for 100% of the DNA in the specimen, the change of the fluorescence intensity per unit temperature change from the DNA intercalator decreases gradually. In addition, when the temperature of the specimen is raised, the amount of increase and the amount of decrease of the change of the fluorescence intensity per unit temperature change become larger as the proportion of linear DNA becomes higher and the proportion of circular DNA becomes lower.

Accordingly, when the temperature of the specimen is raised from the vicinity of the temperature range in which linear DNA dissociates or a temperature equal to or lower than the temperature range and the change of the fluorescence intensity per unit temperature change is used as the transition change signal, it indicates that linear DNA is included in the specimen if a temperature range in which at least one of the amount of increase and the amount of decrease of the change of the fluorescence intensity per unit temperature change is equal to or greater than a predetermined value is included (that is, if a drastic increase and/or a drastic decrease of the change occurs).

When the temperature of the specimen is raised and the change of the fluorescence intensity per unit temperature change is used as the transition change signal, it indicates that linear DNA is not included in the specimen and only circular DNA is included in the specimen if a temperature range in which at least one of the amount of increase and the amount of decrease of the change of the fluorescence intensity per unit temperature change is equal to or greater than a predetermined value is not included (that is, if a drastic increase and/or a drastic decrease of the change does not occur).

When the temperature of the specimen is raised and the change of the fluorescence intensity per unit temperature change is used as the transition change signal, it indicates that circular DNA is not included in the specimen and only linear DNA is included in the specimen if at least one of the amount of increase and the amount of decrease of the change of the fluorescence intensity per unit temperature change is equal to the amount of increase or the amount of decrease when linear DNA accounts for 100%.

When the temperature of the specimen is raised and the change of the fluorescence intensity per unit temperature change is used as the transition change signal, the smaller at least one of the amount of increase and the amount of decrease of the change of the fluorescence intensity per unit temperature change indicates the higher proportion of circular DNA and the lower proportion of linear DNA in the DNA included in the specimen, and the larger at least one of the amount of increase and the amount of decrease of the change of the fluorescence intensity per unit temperature change indicates the higher proportion of linear DNA and the lower proportion of circular DNA in the DNA included in the specimen.

The "at least one of the amount of increase and the amount of decrease of the change of the fluorescence intensity per unit temperature change" may be both of the amount of increase and the amount of decrease of the change of the fluorescence intensity per unit temperature change.

When a change of fluorescence intensity per unit temperature change is used as the transition change signal, the amount of increase and the amount of decrease of the change may be, for example, the amount of increase and the amount of decrease of the change immediately before the change of fluorescence intensity per unit temperature change in a melting curve becomes equal to or greater than a predetermined value (in a slightly higher or lower temperature range or at a unit temperature), the amount of increase and the amount of decrease of the change of the fluorescence intensity per unit temperature change in the same temperature range of a sample in which only circular DNA is included as the DNA (linear DNA is not included) because the change undulates less, or the amount of increase and the amount of decrease of the change of the fluorescence intensity per unit temperature change in an arbitrary temperature range not crossing a peak of the melting curve illustrated in FIG. 2 or an area in which the melting curve decreases drastically thereafter (preferably, a temperature range lower than the peak or a temperature range lower than the area).

In the estimation method according to the present invention, "linear DNA is not included (not contained)" means that the content of linear DNA in the specimen is lower than a detection limit of the estimation method, and "circular DNA is not included (not contained)" means that the content of circular DNA in the specimen is lower than a detection limit of the estimation method.

The "predetermined value" when the temperature of the specimen is raised can be appropriately determined, for example, such that the amount of increase and/or the amount of decrease acquired in a sample in which the proportion of circular DNA is 100% is not equal to or greater than the predetermined value and the amount of increase and the amount of decrease of the change in a sample in which the proportion of linear DNA is 100% is equal to or greater than the predetermined value after having ascertained the amount of increase and/or the amount of decrease of the change of the fluorescence intensity per unit temperature change when the sample in which the proportion of circular DNA is 100% and the sample in which the proportion of linear DNA is 100% are heated under the presence of a DNA intercalator.

Regarding the "predetermined value," for example, a discrimination expression may be prepared using a known statistical analysis method in the corresponding technical field, and a threshold value may be calculated from a measured value of the amount of increase and the amount of decrease of the accumulated change using this expression.

One or more of whether there is linear DNA and/or circular DNA, proportions thereof, and amounts thereof may be automatically determined using machine learning on the basis of the measured value of the amount of increase and/or the amount of decrease of the change of the fluorescence intensity per unit temperature change.

A temperature range in which the change increases or decreases drastically is near the temperature range in which linear DNA dissociates, and an example thereof is a range of 80°C to 93°C or a range of 80°C to 90°C though it varies slightly depending on the GC content or the length of the DNA. Drastic increase (drastic decrease of the negative differential value vs temperature -dF/dT) of the change of the fluorescence intensity (differential value vs temperature, dF/dT) often occurs in the temperature range of 85°C to 93°C, and drastic decrease (drastic increase of the negative differential value vs temperature -dF/dT) of the change of the fluorescence intensity often occurs in the temperature range of 80°C to 85°C. Accordingly, it is particularly preferable to ascertain the amount of increase and/or the amount of decrease of the change of the fluorescence intensity per unit temperature change in such a temperature range or a temperature range close thereto.

When the temperature of the specimen is lowered and there is linear DNA in the specimen, a change of fluorescence intensity per unit temperature change dF/dT from a DNA intercalator increases or decreases drastically in the vicinity of a temperature range in which linear DNA dissociates. On the other hand, when circular DNA accounts for 100% of the DNA in the specimen, the change of the fluorescence intensity per unit temperature change from the DNA intercalator increases gradually.

Accordingly, when the temperature of the specimen is lowered from the vicinity of the temperature range in which linear DNA dissociates or a temperature equal to or higher than the temperature range and the change of the fluorescence intensity per unit temperature change is used as the transition change signal, it indicates that linear DNA is included in the specimen if a temperature range in which at least one of the amount of increase and the amount of decrease of the change of the fluorescence intensity per unit temperature change is equal to or greater than a predetermined value is included (that is, if a drastic increase and/or a drastic decrease of the change occurs).

When the temperature of the specimen is lowered and the change of the fluorescence intensity per unit temperature change is used as the transition change signal, it indicates that linear DNA is not included in the specimen and only circular DNA is included in the specimen if a temperature range in which at least one of the amount of increase and the amount of decrease of the change of the fluorescence intensity per unit temperature change is equal to or greater than a predetermined value is not included (that is, if a drastic increase and/or a drastic decrease of the change does not occur).

When the temperature of the specimen is lowered and the change of the fluorescence intensity per unit temperature change is used as the transition change signal, it indicates that circular DNA is not included in the specimen and only linear DNA is included in the specimen if at least one of the amount of increase and the amount of decrease of the change of the fluorescence intensity per unit temperature change is equal to the amount of increase or the amount of decrease when linear DNA accounts for 100%.

When the temperature of the specimen is lowered and the change of the fluorescence intensity per unit temperature change is used as the transition change signal, the smaller at least one of the amount of increase and the amount of decrease of the change of the fluorescence intensity per unit temperature change indicates the higher proportion of circular DNA and the lower proportion of linear DNA in the DNA included in the specimen, and the larger at least one of the amount of increase and the amount of decrease of the change of the fluorescence intensity per unit temperature change indicates the higher proportion of linear DNA and the lower proportion of circular DNA in the DNA included in the specimen.

The "at least one of the amount of increase and the amount of decrease of the change of the fluorescence intensity per unit temperature change" may be both of the amount of increase and the amount of decrease of the change of the fluorescence intensity per unit temperature change.

The "predetermined value" when the temperature of the specimen is lowered can be appropriately determined, for example, such that the amount of increase and/or the amount of decrease acquired in a sample in which the proportion of circular DNA is 100% is not equal to or greater than the predetermined value and the amount of increase and the amount of decrease of the change in a sample in which the proportion of linear DNA is 100% is equal to or greater than the predetermined value after having ascertained the amount of increase and/or the amount of decrease of the change of the fluorescence intensity per unit temperature change when the sample in which the proportion of circular DNA is 100% and the sample in which the proportion of linear DNA is 100% are heated under the presence of a DNA intercalator. The temperature range in which the change of the fluorescence intensity increases and/or decreases drastically is the same as described above.

As described above, for example, a discrimination expression may be prepared using a known statistical analysis method in the corresponding technical field, and a threshold value may be calculated from a measured value of the amount of increase and the amount of decrease of the accumulated change using this expression.

One or more of whether there is linear DNA and/or circular DNA, proportions thereof, and amounts thereof may be automatically determined using machine learning on the basis of the measured value of the amount of increase and/or the amount of decrease of the change of the fluorescence intensity per unit temperature change.

When the change of the fluorescence intensity per unit temperature change is used as the transition change signal, it is preferable to prepare a melting curve in which the vertical axis is set to dF/dT or -dF/dT and the horizontal axis is set to the temperature as will be described later in examples. By preparing the melting curve, it is possible to easily ascertain the magnitude of a positive peak (the amount of increase) or the magnitude of a negative peak (the amount of decrease) of the change of the fluorescence intensity per unit temperature change in the vicinity of the temperature range in which linear DNA dissociates.

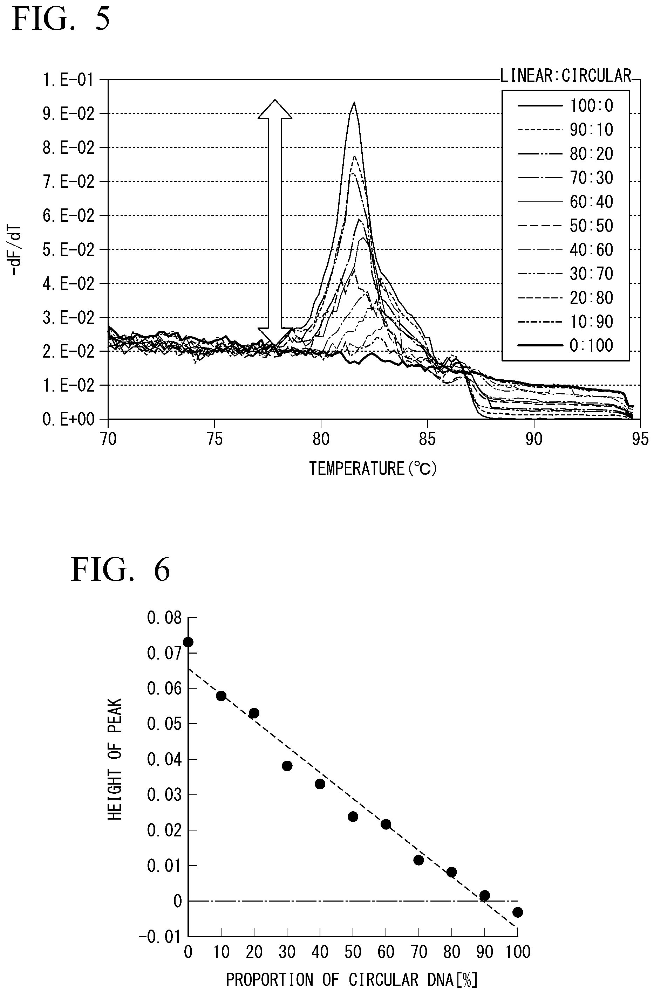

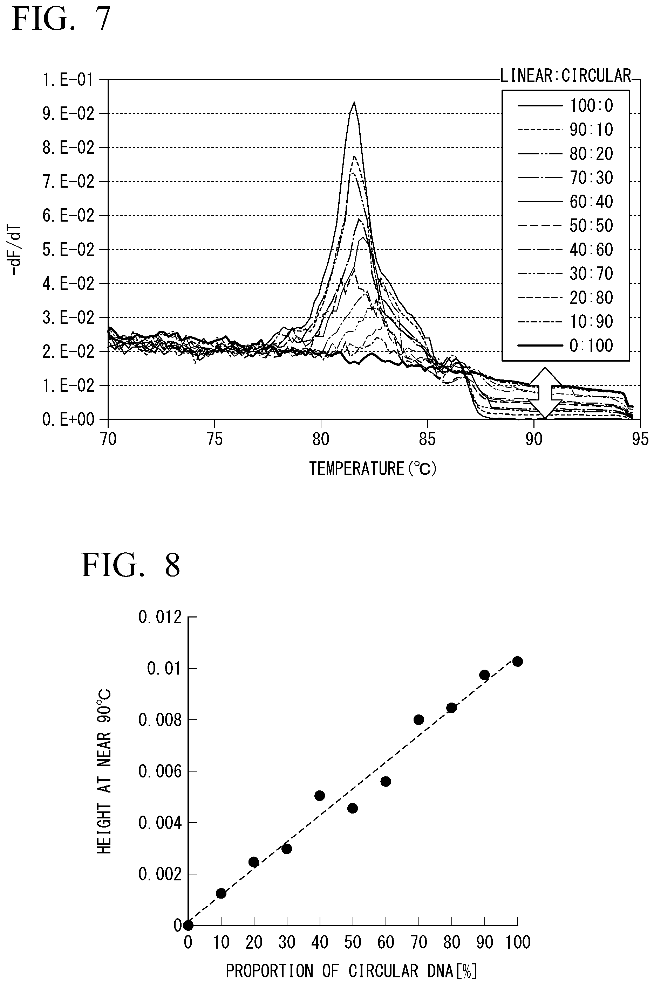

When the change of the fluorescence intensity per unit temperature change is used as the transition change signal, it is preferable to prepare a calibration curve by heating or cooling a sample including DNA in which the proportion of linear DNA and the proportion of circular DNA are determined, acquiring the amount of increase and/or the amount of decrease of the change of the fluorescence intensity per unit temperature change, and plotting the proportion of linear DNA and/or the proportion of circular DNA with respect to the acquired amount of increase and/or the acquired amount of decrease. By comparing the amount of increase and/or the amount of decrease of the change of the fluorescence intensity per unit temperature change in the specimen including DNA with the calibration curve, it is possible to easily estimate whether there is linear DNA and/or circular DNA, the proportion of linear DNA and/or the circular DNA, and the amount of the linear DNA and/or the circular DNA and to perform estimation with higher accuracy than that in the method using electrophoresis in the related art (see FIGS. 6 and 8 which will be described later in detail in examples).

In a plot for drawing the calibration curve, a plurality of samples in which the proportions of linear DNA and/or circular DNA are determined may be used, but it is possible to easily prepare the calibration curve, for example, by heating or cooling a sample in which the proportion of circular DNA is 100% (the proportion of linear DNA is 0%) and a sample in which the proportion of linear DNA is 100% (the proportion of circular DNA is 100%), plotting the ratio (100% or 0%) between the acquired amount of increase or the acquired amount of decrease of the change of the fluorescence intensity per unit temperature change, and connecting a point (coordinates) at 0% and a point at 100%. The sample in which the proportion of linear DNA is 100% can be obtained, for example, by treating the sample in which the proportion of circular DNA is 100% using a restriction enzyme.

An example in which a calibration curve indicating a relationship between the amount of decrease of the change of the fluorescence intensity per unit temperature change (-dF/dT) and the proportion of linear DNA when the change of the fluorescence intensity per unit temperature change is detected as the transition change signal by raising the temperature of the specimen is prepared will be described below.

First, a melting curve in which the vertical axis is set to -dF/dT is prepared for the sample in which the proportion of circular DNA is 100%, and the temperature at which the value of - dF/dT of the melting curve is 0 is identified. For example, in a melting curve illustrated in FIG. 5 which will be described later, the value of -dF/dT decreases drastically to 0 in the vicinity of 88°C. Thereafter, a melting curve of one or more samples in which the proportion of linear DNA is determined is prepared, the proportion of linear DNA and/or circular DNA with respect to the amount of decrease from -dF/dT in the sample in which the proportion of circular DNA is 100% in the temperature range up to -dF/dT of the samples in an arbitrary temperature range in the temperature range from the temperature at which -dF/dT is 0 to 95°C (or 90°C) is plotted, and a calibration curve can be prepared and used to estimate one or more of whether there is linear DNA and/or circular DNA in the specimen, the proportion thereof, and the amount thereof.

When a calibration curve is not drawn and one or more of whether there is linear DNA and/or circular DNA in the specimen, the proportion thereof and the amount thereof are estimated from the melting curve, it is possible to perform estimation with high accuracy by comparing the amount of increase or decrease in the change in the melting curve with that of a sample for which the proportions of linear DNA and circular DNA are already known, obtained upon raising or lowering the temperature.

As will be described later in Experimental Example 3, the melting curve of the sample in which the proportion of circular DNA is 100% extends substantially linearly, and -dF/dT for the sample in which the proportion of linear DNA is 100% decreases to 0 in the temperature range of 85°C to 95°C.

Accordingly, by comparing the height position (-dF/dT) of the melting curve for the sample in which the proportion of circular DNA is 100% extending substantially linearly in the temperature range of 85°C to 95°C (for example, the temperature range of 90°C to 93°C), the height position at which dF/dT is 0, and the height position at which the melting curve of the specimen for estimating the proportion of linear DNA and/or circular DNA, it is possible to accurately estimate the proportion of linear DNA and/or circular DNA.

That is, it can be estimated that the proportion of linear DNA becomes lower and the proportion of circular DNA becomes higher as the value of -dF/dT of the melting curve of the specimen in which the proportion of linear DNA and/or circular DNA is to be estimated in the temperature range of 85°C to 95°C becomes closer to the value of -dF/dT in the melting curve of the sample in which the proportion of circular DNA is 100%, and the proportion of linear DNA becomes higher and the proportion of circular DNA becomes lower as the value of -dF/dT becomes closer to 0.

In this case, for example, a difference between the value of -dF/dT of the specimen and the value of -dF/dT of the sample in which the proportion of circular DNA is 100% in the temperature range of 85°C to 95°C (or a temperature range of 90°C to 93°C) at which the value of -dF/dT decreases drastically can also be understood as the amount of decrease of the change, and it can be estimated that the larger this amount of decrease, the higher the proportion of linear DNA and the lower the proportion of circular DNA. As will be described below, the melting curve for the sample in which the proportion of circular DNA is 100% can be predicted from the melting curve for the specimen in which the proportion of linear DNA and/or circular DNA is to be estimated.

FIG. 2 is a graph illustrating a virtual melting curve in a sample in which the proportion of circular DNA is 100% which is prepared on the basis of a melting curve of a sample including linear DNA.

The melting curve for the sample in which the proportion of circular DNA is 100% extends substantially linearly as described above, and thus when the temperature is raised and the change of the fluorescence intensity per unit temperature change which is the transition change signal is detected, a virtual line A obtained by extending a line in a temperature range (for example, 70°C to 75°C) not covering a peak in the melting curve for the specimen in which whether there is linear DNA and/or circular DNA or the proportion thereof is to be estimated linearly to a high temperature side while maintaining a slope thereof is substantially the same as the melting curve for the sample in which the proportion of circular DNA is 100%. Accordingly, by comparing the height position (-dF/dT) of the virtual line A in the temperature range of 85°C to 93°C (for example, the temperature range of 90°C to 93°C) in which the value of -dF/dT has decreased drastically, a height position (-dF/dT) of the melting curve for the specimen in which the proportion of linear DNA and/or circular DNA is to be estimated, and the height position at which the value of -dF/dT is 0, it is possible to estimate one or more of whether there is linear DNA and/or circular DNA, the proportion thereof, and the amount thereof. Accordingly, it is not necessary to prepare a sample in which the proportion of circular DNA is 100% and a sample in which the proportion of linear DNA is 100% as a comparison target.

It is preferable that the "sample in which the proportions of linear DNA and circular DNA are determined" be the same as a specimen in which whether there is linear DNA and/or circular DNA or the proportion thereof is to be estimated in at least one of the length of DNA, the GC content, the nucleotide sequence, and the DNA concentration, and it is more preferable that the length of DNA, the GC content, the nucleotide sequence, and the DNA concentration be the same as those in the specimen in which whether there is linear DNA and/or circular DNA or the proportion thereof is to be estimated. For example, since the DNA concentration can be calculated on the basis of an absorbance (for example, 260 nm) of the specimen, the concentration of the "sample in which the proportions of linear DNA and circular DNA are determined" calculated from the absorbance and the concentration of the specimen may be diluted such that both are the same in advance and may be heated or cooled. After the melting curve of one has been shifted vertically such that the vertical position (the value of -dF/dT) of an end on a low temperature side is the same between one melting curve and the other melting curve instead of diluting one, the proportion of linear DNA may be estimated by comparing one and the other.

On the other hand, the amount of linear DNA and/or circular DNA in the specimen can be estimated by multiplying the proportion of linear DNA and/or circular DNA estimated on the basis of the change of the fluorescence intensity per unit temperature change by the amount of DNA in the specimen, the amount of DNA can also be directly estimated without calculating the proportion on the basis of a relationship between the amount of increase and/or the amount of decrease of the change of the fluorescence intensity per unit temperature change and the amount of linear DNA or circular DNA, and the present invention is not limited thereto.

When the amount of linear DNA and/or circular DNA in the specimen is directly estimated on the basis of the relationship between the amount of increase and/or the amount of decrease of the change of the fluorescence intensity per unit temperature change and the amount of linear DNA or circular DNA, for example, the amount of DNA may be estimated using a calibration curve obtained by plotting the relationship between the amount of increase and/or the amount of decrease of the change of the fluorescence intensity per unit temperature change and the amount of linear DNA or circular DNA in the coordinate plane, or the amount of DNA may be estimated by setting the relationship as a lookup table or a mapping table and acquiring the amount of linear DNA or circular DNA corresponding to the amount of increase and/or the amount of decrease of the change of the fluorescence intensity per unit temperature change.

In this way, when the amount of linear DNA or circular DNA is directly estimated without using the proportions thereof, the total amount of DNA in the specimen may be adjusted in advance to be the same as the total DNA in the sample used to prepare the calibration curve, the lookup table, or the like before the estimation, and then the estimation may be performed.

While the estimation method when a change of fluorescence intensity per unit temperature change is used as the transition change signal has been described above in detail, it is also possible to similarly estimate one or more of whether there is linear DNA and/or circular DNA in a specimen, the proportion thereof, and the amount thereof even when a measurement result obtained by differential scanning calorimetry (DSC) is used as the transition change signal.

For example, when an absorbance is used as the transition change signal and DNA transitions from double strands to a single strand, the absorbance of ultraviolet (for example, 260 nm) increases by about 40%, and thus it is possible to estimate one or more of whether there is linear DNA and/or circular DNA in the specimen, the proportion thereof, and the amount thereof from whether there is a drastic decrease and/or a drastic increase of a change of the absorbance per unit temperature change or the amount of increase and the amount of decrease of the change similarly to the change of the fluorescence intensity per unit temperature change. When the measurement result using differential scanning calorimetry is used as the transition change signal, it is possible to estimate one or more of whether there is linear DNA and/or circular DNA in the specimen, the proportion thereof, and the amount thereof from a difference in thermophysical property between linear DNA and circular DNA.

With the estimation method according to the present embodiment, since a temperature of a specimen is raised or lowered, a signal varying with transition (shift) between a double-stranded state and a single-stranded state of DNA is detected, and one or more of whether there is linear DNA and/or circular DNA in the specimen, the proportion thereof, and the amount thereof can be estimated on the basis of the signal, it is possible to simply estimate one or more of whether there is linear DNA and/or circular DNA in the specimen, the proportion thereof, and the amount thereof.

With the estimation method according to the embodiment, about 30 minutes is required for obtaining a result using the method based on electrophoresis according to the related art, but it is possible to estimate one or more of whether there is linear DNA and/or circular DNA in the specimen, the proportion thereof, and the amount thereof for a short time of about 10 minutes.

For example, when a specimen includes a plasmid vector DNA, the proportion of linear DNA estimated using the estimation method according to the present embodiment is high, and the proportion of circular DNA is low, it is predicted that the phenotype transformation efficiency is low, and thus measures such as changing a purification method of the plasmid vector DNA and curbing physical shearing of circular DNA can be taken.

With the estimation method according to the present embodiment, the absolute amount of linear DNA may be estimated by multiplying the proportion of linear DNA acquired using the estimation method according to the present embodiment by the absolute amount of DNA including circular DNA and linear DNA in the specimen, or the absolute amount of circular DNA may be estimated from the absolute amount of DNA as a whole and the proportion of circular DNA obtained using the estimation method according to the embodiment or the absolute amount of linear DNA. For example, when the absolute amount of DNA included in a specimen is 100 ng and the proportion of circular DNA therein is estimated to be 80% (0.8) using the estimation method, the absolute amount of circular DNA is estimated to be 80 ng which is a product thereof.

While an exemplary embodiment has been described above in detail, the present invention is not limited to the embodiment and can be modified in various forms within the scope of the inventions described in the appended claims.

### [Examples]

The present invention will be described below in conjunction with examples, and the present invention is not limited to the examples.

### [Experimental Example 1]

In the present experiment, a sample in which the proportion of linear DNA is 100% was prepared from a sample in which the proportion of circular DNA is 100%, and a change of fluorescence intensity (fluorescence luminance) and a melting curve were ascertained by heating the sample in which the proportion of circular DNA is 100% and the prepared sample.

### (Preparation of sample)

2 µg of a single (with the same sequence and length of DNA) plasmid with one recognition site of EcoRI was treated with EcoRI (Fast Digest EcoRI, Thermo Fisher Scientific), and a sample in which the proportion of linear DNA is 100% was prepared. Since the reaction volume was 20 µL, the final concentration was 100 ng/µL.

In order to prepare a sample in which the proportion of circular DNA is 100% under the same conditions as linear DNA, a system to which pure water was added was separately prepared instead of EcoRI.

### (Measurement of fluorescence intensity)

0.4 µL of a DNA intercalator (Lamp Fluorescent Dye, New England Biolabs) was added to 19.6 µL of the sample prepared in the aforementioned order, and the resultant was provided to measure fluorescence intensity accompanying heating. A real-time PCR system (QuantStudio 3, Thermo Fisher Scientific) was used to measure the fluorescence intensity.

As the heating condition, the resultant was heated to 50°C at 1.6 °C/sec, was maintained at 50°C for 1 minute, and was additionally heated to 95°C at 0.1 °C/sec.

### (Data analysis)

Numerical values output from the real-time PCR system were used for a graph of a change of the fluorescence intensity and the melting curve without any change.

FIG. 3 is a graph illustrating a relationship between the fluorescence intensity and the temperature when a sample in which the proportion of circular DNA is 100% and a sample in which the proportion of linear DNA is 100% are heated. FIG. 4 is a graph illustrating a melting curve when a sample in which the proportion of circular DNA is 100% and a sample in which the proportion of linear DNA is 100% are heated.

As illustrated in FIG. 3, it could be seen that fluorescence intensity (fluorescence luminance) generated from a DNA intercalator is lowered with raising of the temperature and DNA dissociates from a double-stranded state to a single-stranded state with raising of the temperature.

In the sample in which the proportion of circular DNA is 100%, the change of the fluorescence intensity is slow, and -dF/dT which is a change of the fluorescence intensity per unit temperature change illustrated in FIG. 4 changes slowly.

On the other hand, in the sample in which the proportion of linear DNA is 100%, it was ascertained that the fluorescence intensity decreased rapidly in the temperature range of 80°C to 85°C as illustrated in FIG. 3 and -dF/dT (a negative differential value) which is a change of the fluorescence intensity per unit temperature change increased drastically as illustrated in FIG. 4.

In the sample in which the proportion of linear DNA is 100%, it was hinted that -dF/dT decreased drastically and rapidly in the temperature range of 85°C to 90°C as illustrated in FIG. 4.

### [Experimental Example 2]

In the present experiment, a plurality of samples in which the ratio between linear DNA and circular DNA was varied were prepared and heated, and melting curves were prepared. The total amount of DNA was the same between the samples. Thereafter, a calibration curve was prepared by plotting the proportion of circular DNA with respect to a height of a peak (the amount of increase) of a negative differential value vs temperature.

### (Preparation of sample)

Specifically, first, in the same order as in Experimental Example 1, a sample in which the proportion of circular DNA is 100% and a sample in which the proportion of linear DNA is 100% were prepared, and samples in which the proportion of circular DNA was 0%, 10%, 20%, 30%, 40%, 50%, 60%, 70%, 80%, 90%, and 100% were prepared by mixing both prepared samples at arbitrary ratios or using one sample without any change without mixing the prepared samples . For example, when the sample in which the proportion of circular DNA was 20% was prepared, 4 µL of the sample in which the proportion of circular DNA is 100% was added to 16 µL of the sample in which the proportion of linear DNA is 100%. A sample in which the proportion of circular DNA was 35% was separately prepared as a sample in which the ratio is unknown.

### (Measurement and analysis of fluorescence intensity)

Fluorescence intensity was measured similarly to Experimental Example 1. Thereafter, a background value which is fluorescence intensity in a sample not including DNA was subtracted from the fluorescence intensity output from the real-time PCR system.

Subsequently, a moving average in which the number of sections was 5 (one section which is a unit temperature change: 0.2°C) was calculated for the acquired value. That is, a moving average of an n-th term in a1, a2, a3, ... was mₐₙ=(aₙ₋₂+aₙ₋₁+aₙ+aₙ₊₁+aₙ₊₂)/5. Thereafter, the moving average was normalized such that an initial value (T=value at 50°C) of the fluorescence intensity was 1. Finally, the rate of change (the amount of change) D was calculated using Expression (i), and a simulative melting curve was calculated. The rate of change of the n-th term was defined as Dn', the temperature was defined as Tₙ, and the normalized fluorescence intensity was defined as Fₙ. $Dn = \left({F}_{n + 1}-{F}_{n}\right) / \left({T}_{n + 1}-{T}_{n}\right)$ (i)

In the graph prepared in the aforementioned order, a height of a peak (the amount of increase) of a negative differential value which is a change of the fluorescence intensity per unit temperature change vs temperature was calculated by subtracting an average value in the range of 74°C to 76°C from a maximum value of the peak. The calibration curve for the height of the peak with respect to the proportion of circular DNA was prepared.

FIG. 5 is a graph illustrating melting curves which are prepared by heating a plurality of samples in which the ratio between linear DNA and circular DNA was varied. In FIG. 5, the amount of increase by which the change of the fluorescence intensity per unit temperature change increases drastically is indicated by a bidirectional arrow.

FIG. 6 is a calibration curve which is prepared by plotting the proportion of circular DNA with respect to the amount of increase of the change of the fluorescence intensity per unit temperature change when a plurality of samples in which the ratio between linear DNA and circular DNA was varied were heated.

As illustrated in FIG. 5, a peak (a quick increase in change) of the change -dF/dT of the fluorescence intensity per unit temperature change was seen in the temperature range of 80°C to 85°C. The magnitude of the peak (the amount of increase) indicated by the bidirectional arrow in FIG. 5 became larger as the proportion of linear DNA became higher (that is, as the magnitude of the peak became larger, the proportion of circular DNA became lower).

The magnitude of a peak in the sample in which the ratio was unknown was applied to the analytic curve illustrated in FIG. 6, and the proportion of circular DNA in the unknown sample was estimated to be 38.3%. Since the actual proportion of circular DNA in the unknown sample was 35%, it was hinted that the proportion of circular DNA and the proportion of linear DNA could be estimated with considerably high accuracy from the amount of increase at a position at which the change of the fluorescence intensity per unit temperature change increased drastically.

### [Experimental Example 3]

In the present experiment, a correlation between the amount of decrease of the value of - dF/dT in the vicinity of 90°C and the proportion of circular DNA in the melting curve of FIG. 5 obtained in Experimental Example 2. Specifically, a calibration curve was prepared by plotting the proportion of circular DNA with respect to the height (the amount of decrease, the value of - dF/dT) in the temperature range of 89°C to 90°C.

FIG. 7 is a graph illustrating a height of the change (the amount of decrease) of the fluorescence intensity per unit temperature change in a temperature range of 89°C to 90°C in the melting curve illustrated in FIG. 5 using an arrow. FIG. 8 is a calibration curve prepared by plotting the proportion of circular DNA with respect to the height of the change of the fluorescence intensity per unit temperature change in the temperature range of 89°C to 90°C.

As illustrated in FIG. 7, it was ascertained in the sample including linear DNA that the change -dF/dT of the fluorescence intensity per unit temperature change decreased drastically in the temperature range of 85°C to 90°C. As illustrated in FIG. 8, it was found that the height of the change of the fluorescence intensity per unit temperature change in the temperature range near 90°C had a strong correlation with the proportion of circular DNA. In other words, it was found that the amount of decrease in the drastic decrease of the change of the fluorescence intensity per unit temperature change in the temperature range near 90°C was proportional to the proportion of linear DNA. From this result, it was hinted that the proportions of circular DNA and linear DNA could be accurately estimated from the height of the change of the fluorescence intensity per unit temperature change in the vicinity of 90°C when the specimen was heated or the amount of decrease of the change.

According to the present invention, since one or more of whether there is linear DNA and/or circular DNA, the proportion, and the amount can be estimated by raising or lowering the temperature of a specimen and detecting a signal varying with transition between a double-stranded state and a single-stranded state of DNA, the estimation method is industrially applicable.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. An estimation method comprising:
raising or lowering a temperature of a specimen including DNA and detecting a signal varying with transition between a double-stranded state and a single-stranded state of DNA; and
estimating at least one of whether there is at least one of linear DNA and circular DNA in the specimen, a proportion of at least one of linear DNA and circular DNA in the DNA in the specimen, and an amount of at least one of linear DNA and circular DNA in the specimen on the basis of the signal.

2. The estimation method according to claim 1, wherein the signal is based on fluorescence intensity from a DNA intercalator that emits fluorescence when the DNA intercalator is inserted between double strands of DNA.

3. The estimation method according to claim 2, wherein the signal is change of the fluorescence intensity per unit temperature change, and
wherein the signal is detected by raising the temperature of the specimen.

4. The estimation method according to claim 3, wherein whether there is circular DNA or a proportion thereof is estimated on the basis of the signal.

5. The estimation method according to claim 4, wherein, when a drastic increase and a drastic decrease of the change do not occur, it indicates that only circular DNA is included in the specimen.

6. The estimation method according to claim 3, wherein, when at least one of a drastic increase and a drastic decrease of the change occurs, it indicates that linear DNA is included in the specimen.

7. The estimation method according to claim 6, wherein, when a temperature range in which at least one of the amount of increase and the amount of decrease of the change is equal to or higher than a predetermined value is included, it indicates that linear DNA is included in the specimen, and
wherein, when a temperature range in which at least one of the amount of increase and the amount of decrease of the change is equal to or higher than the predetermined value is not included, it indicates that circular DNA is included and linear DNA is not included in the specimen.

8. The estimation method according to claim 7, wherein the amount of increase is the amount of increase of the change in a range of 85°C to 93°C, and
wherein the amount of decrease is the amount of decrease of the change in a range of 80°C to 85°C.

9. The estimation method according to claim 8, wherein a larger amount of increase or a larger amount of decrease of the change indicates a higher proportion of linear DNA and a lower proportion of circular DNA in the DNA in the specimen.

10. The estimation method according to claim 1, wherein the DNA included in the specimen is at least one kind of DNA selected from a group consisting of plasmid DNA, mitochondrial DNA, eccDNA, and ecDNA.

11. An estimation method of estimating an absolute amount of linear DNA in the specimen by multiplying an amount of DNA included in the specimen by the proportion of linear DNA estimated using the estimation method according to any one of claims 1 to 10.

12. An estimation method of estimating an absolute amount of circular DNA in the specimen by multiplying an amount of DNA included in the specimen by the proportion of circular DNA estimated using the estimation method according to any one of claims 1 to 10.
